# EUROPEAN PATENT SPECIFICATION

(11) **EP 3 986 854 B1**
(45) Date of publication and mention of the grant of the patent: **02.08.2023**
(21) Application number: 20733427.7
(22) Date of filing: 18.06.2020
(51) Int. Cl.: C07C 67/29

(54) **METHOD FOR PRODUCING GLYCEROL MONO(METH)ACRYLATE**
VERFAHREN ZUR HERSTELLUNG VON GLYCERINMONO(METH)ACRYLAT
PROCÉDÉ DE PRODUCTION DE MONO(MÉTH)ACRYLATE DE GLYCÉROL

(30) Priority: 21.06.2019 EP 19181573
(43) Date of publication of application: 27.04.2022
(73) Proprietor: Evonik Operations GmbH, 45128 Essen (DE)
(72) Inventor: BESTGEN, Sebastian, 65760 Eschborn (DE); CASPARI, Maik, 64665 Alsbach-Hähnlein (DE); SCHÜTZ, Thorben, 64665 Alsbach-Hähnlein (DE); BLEITH, Tim, 55131 Mainz (DE)
(74) Representative: Evonik Patent Association
(86) International application number: PCT/EP2020/066865
(87) International publication number: WO 2020/254460

(56) References cited:
- DE-A1- 10 308 504
- SUERVY C. O. SOUSA ET AL: "Microwave-promoted morita-baylis-hillman reactions: efficient synthesis of new monoacylglycerols (MAGs) as potential anti-parasitic compounds", JOURNAL OF THE BRAZILIAN CHEMICAL SOCIETY, vol. 22, 1 January 2011 (2011-01-01), pages 1634-1643, XP055630834, São Paulo; BR ISSN: 0103-5053, DOI: 10.1590/S0103-50532011000900003

## Description

### Field of the invention

The invention relates to an anhydrous method for producing glycerol mono(meth)acrylate by reacting 2,2-dimethyl-1,3-dioxolan-4-ylmethyl (meth)acrylate with methanol.

### Prior art

Glycerol mono(meth)acrylate can be used for various industrial applications, for example as a monomer for producing polymers for coating purposes, varnishes, paints, adhesives or contact lenses, as well as in the field of oil-based lubricants and slip additives.

For many applications it is critical that glycerol mono(meth)acrylate is in the form of high-purity monomer since even trace impurities can firstly result in negative effects during storage of the monomer and these secondly also impair the quality of the polymer to be produced.

Particularly undesirable impurities in monomers are those which may act as crosslinkers in a polymerization reaction since the presence of crosslinkers during a polymerization reaction impairs the formation of linear polymers. Even traces of acids considerably reduce the stability of the monomer.

Glycerol mono(meth)acrylate can, in principle, be obtained by various routes.

Firstly, glycerol can be esterified directly with acetylating reagents (e.g. methacrylic acid, methacrylic anhydride, methacryloyl halides). For example, WO 2018/031373 describes the preparation of glycerol monomethacrylate (GMMA) from glycerol and an excess of methacrylic acid in the presence of amberlyst. However, in this case, low selectivity is problematic.

More selective with respect to monofunctionalization is the mostly acid-catalysed epoxide and acetonide cleavage of glycidyl methacrylate or 2,2-dimethyl-1,3-dioxolan-4-ylmethyl methacrylate (IPGMA) in aqueous medium to give GMMA, wherein the cleavage of the harmful to health glydicyl methacrylate leads to the formation of undesired by-products (J. Appl. Polym. Sci., 135, 46579) or requires a laborious column chromatographic purification (Macromolecules 2018, 51, 18, 7396-7406).

The preparation of GMMA by hydrolysis of IPGMA is described, for example, in GB 852 384, wherein the hydrolysis is carried out in the presence of dilute aqueous solutions of strong mineral acids.

In US 2010/249352, the acetonide is cleaved in aqueous medium by addition of an acid, and is then neutralized with a weak base. The water is removed in a laborious process of multiple distillation on a thin-film evaporator.

The preparation of GMMA from IPGMA is also described in DE102016122755. The preparation is also carried out here in aqueous medium in the presence of amberlyst and with simultaneous feed of an O₂/N₂ gas stream.

GMMA can also be obtained via lipase-catalysed transacylation (J. Mol. Cat. B: Enzymatic 2010, 62, 80-89), which requires utilization of enzymes and long reaction times (120 h) and has low conversions.

The publication from Suervy C.O. Sousa et al ("Microwave-promoted morita-baylis-hillman reactions: efficient synthesis of new monoacylglycerols (MAGs) as potential anti-parasitic compounds ", Journal of the Brazilian Chemical Society, Vol. 22, January 2011, pages 1634-1643) discloses several protocols for the acetal cleavage of compounds (20), with best results obtained using amberlyst and methanol with or without microwave irradiation (see Table 1, entries 3 and 4).

DE103 08 504 teaches that the cleavage of the acetals/ketals generally takes place under acid catalysis and with the addition of water.

Most of the methods described above have the addition of water in common, which is very difficult to remove from the remaining reaction mixture/product. The consequence is a high residual water content in the product, which has a negative effect on the further use of glycerol mono(meth)methacrylate or can even completely prevent it. In addition, polymerization occurs during the dewatering phase owing to the prolonged thermal stress.

Particularly for the application of glycerol mono(meth)acrylate in the field of oil-based lubricants and slip additives, water can also be included among the particularly undesirable impurities. In the field of lubricants and hydraulic oils - in addition to contamination with solid particles - contamination with water or moisture can be the cause of component failure.

Water may mix with oil in different ways. At low concentrations, it can be dissolved in oil in the liquid phase and thus may not be visually noticeable (solubility in the range of several hundred ppm, material-dependent and age-dependent): It takes the form of a one-phase system. On further increasing the water content, the solubility limit is exceeded and (microscopically) small droplets are formed in the oil, which is known as an emulsion. The water content has in this case exceeded the saturation point, which results in a distinct and undesirable cloudiness of the oil. On further increasing the water content, this eventually results in the formation of two phases, namely a low-oil water phase and a low-water oil phase.

In the sector of lubricants and hydraulic oils, particularly free water and emulsified water have an adverse effect on the material characteristics, for example on the compressibility. Even low levels of impurities in the range of 1% can result in drastic shortening of the service life of the oil. In moving bearings, the formation of a stable oil film is prevented by water contamination. Furthermore, under high temperature and pressure load, there can be spontaneous evaporation of the water (and thus oil), which results in erosive wear. Oil contamination by water further causes foam formation, oil hydrolysis, metal embrittlement, rust and corrosion.

For the use of glycerol mono(meth)acrylate in oil-based systems, the aim is therefore the lowest possible water content in glycerol mono(meth)acrylate, which cannot be achieved for this hydrophilic monomer by means of current synthetic methods.

The object of the invention, therefore, was to provide an improved method for producing glycerol mono(meth)acrylate, by means of which the problems described above with respect to selectivity and by-product formation can be overcome, in which no toxic starting materials have to be used and in which no laborious dewatering step is required. Ideally, the glycerol mono(meth)acrylate product also should not comprise any traces of acid.

By means of this method, a crosslinker-free and the purest possible glycerol mono(meth)acrylate should be obtained without the use of mutagenic substances.

Moreover, the method should be achievable on an industrial scale.

### Summary of the invention

The object is achieved in that glycerol mono(meth)acrylate is produced by a silicate catalysed cleavage of 2,2-dimethyl-1 ,3-dioxolan-4-ylmethyl (meth)acrylate with methanol.

Accordingly, the method relates to a method for producing glycerol mono(meth)acrylate, particularly glycerol monomethacrylate (GMMA), characterized in that 2,2-dimethyl-1,3-dioxolan-4-ylmethyl (meth)acrylate, especially 2,2-dimethyl-1,3-dioxolan-4-ylmethyl methacrylate (IPGMA), is reacted with methanol in the presence of an acidic silicate catalyst,
wherein the silicate is a sheet silicate or a mixture of sheet silicates selected from the group consisting of montmorillonite, kaolinite, hectorite, halloysite and bentonite, and
wherein the reaction is performed in anhydrous manner and in that the product is anhydrous glycerol mono(meth)acrylate.

In this manner, anhydrous glycerol mono(meth)acrylate is obtained without using glycidyl methacrylate that is harmful to health. By avoiding an initial water input to the reaction mixture, a time- and energy-intensive removal of water from the end product is avoided. Also, a prolonged dewatering phase and thus accompanying polymerization of the monomer is prevented.

### Detailed description of the invention

The invention relates to both glycerol monomethacrylate (GMMA) and glycerol monoacrylate (GMA). Accordingly, the term glycerol mono(meth)acrylate, as used in the context of this invention, includes both GMMA and GMA.

The ratio by weight of 2,2-dimethyl-1 ,3-dioxolan-4-ylmethyl (meth)acrylate to methanol in the method according to the invention should be in the range from 1:1 to 1:20, preferably from 1:1 to 1:15 and particularly preferably in the range from 1:1 to 1:4.

For the reaction, both technical-grade methanol and high purity methanol with a purity of 99.9% can be used.

The suitable silicate catalysts is a sheet silicate or a mixture of sheet silicates selected from the group consisting of montmorillonite, kaolinite, hectorite, halloysite and bentonite. Particular preference is given to using acidic sheet silicates and aluminium silicates, such as Tonsil^{®} 312 FF or montmorillonite K10, K30 etc. The catalysts used are particularly particles with the greatest possible specific surface area, particularly with specific surface areas greater than 50 m²/g, preferably with a specific surface area greater than 220 m²/g, and particularly preferably with a specific surface area greater than 320 m²/g.

Compared to homogeneous-catalysed acetonide cleavage of 2,2-dimethyl-1,3-dioxolan-4-ylmethyl (meth)acrylate with p-TsOH (para-toluenesulfonic acid) or the heterogeneous-catalysed acetonide cleavage of 2,2-dimethyl-1,3-dioxolan-4-ylmethyl (meth)acrylate with acidic ion exchange resins (Amberlyst^{®}), an exceptionally low-crosslinker glycerol mono(meth)acrylate product is obtained particularly when using silicate-based catalysts: contamination with glycerol di(meth)acrylate is less than 1%. The product is also virtually free of free glycerol (< 1%), which is also found in greater amounts in conventional synthetic routes.

The reaction is performed in anhydrous manner and the product is anhydrous glycerol mono(meth)acrylate.

The acidic silicate catalyst is used in an amount of 0.5% by weight to 20% by weight, preferably in an amount of 1% by weight to 15% by weight, and especially in an amount of approximately 10% by weight, based on the reaction batch.

Pre-treatment of the catalyst is generally not required.

For stabilizing the starting material and/or product, stabilizers/polymerization inhibitors may be used.

Preferred polymerization inhibitors that can be used include, inter alia, octadecyl-3-(3,5-di-tert-butyl-4-hydroxyphenyl) propionate, phenothiazine, hydroquinone, hydroquinone monomethyl ether, 4-hydroxy-2,2,6,6-tetramethylpiperidinooxyl (TEMPOL), 2,4-dimethyl-6-tert-butylphenol, 2,6-di-tert-butylphenol, 2,6-di-tert-butyl-4-methylphenol, para-substituted phenylenediamines such as for example N,N'-diphenyl-p-phenylenediamine, 1,4-benzoquinone, 2,6-di-tert-butyl-alpha-(dimethylamino)-p-cresol and 2,5-di-tert-butylhydroquinone. These compounds can be used individually or in the form of mixtures and are generally commercially available. The mode of action of the stabilizers is usually that they act as free-radical scavengers for the free radicals that occur in the polymerization. Further details can be found in the relevant technical literature, particularly Römpp-Lexikon Chemie; publisher: J. Falbe, M. Regitz; Stuttgart, New York; 10th edition (1996); keyword "Antioxidantien" and the literature references cited therein.

The total amount of stabilizers used is between 0.0001% by weight and 0.5% by weight, preferably in the weight range between 0.001% by weight and 0.05% by weight.

Preferably, the stabilizers hydroquinone monomethyl ether and hydroxy-2,2,6,6-tetramethylpiperidinooxyl (TEMPOL) are used in combination. The ratio of hydroquinone monomethyl ether to TEMPOL is ideally in the range between 15:1 and 1:1, preferably in the range between 10:1 and 4:1.

In addition, gaseous oxygen can be used for stabilization. This can, for example, be in the form of air, in which the amounts introduced should be adjusted such that the content in the gas phase above the reaction mixture remains below the explosion limit.

The reaction times depend, inter alia, on the selected parameters such as pressure and temperature. In general, however, they are in the range from 1 to 65 hours, preferably 5 to 24 hours and especially preferably 5 to 22 hours. In the continuous processes, the residence times are generally in the range of 5 to 24 hours, preferably 5 to 22 hours.

The reaction can be carried out preferably while stirring, wherein the stirring speed is in the range of 50 to 2000 rpm and preferably in the range of 100 to 500 rpm.

The reaction is ideally carried out at standard pressure. The reaction temperature is between 20°C and 80°C, preferably between 40°C and 70°C.

The method according to the invention can be operated industrially, specifically both in continuous/semi-continuous mode and batchwise mode.

In comparison to the methods known from the literature, glycerol mono(meth)acrylate is obtained by the method according to the invention not only in anhydrous, but also in highly pure form.

The glycerol mono(meth)acrylate obtained by the method according to the invention can be used in applications which require a low residual water content or even the complete absence of water. In particular, it can be used in oil-based systems in the sector of lubricants and hydraulic oils, and in polymerization reactions in liquid and hydrophobic media.

The following examples illustrate the method according to the invention without these being limited thereto.

### Examples

| | |
|---|---|
| **Apparatus:** | 500 ml stirring apparatus, air inlet, Büchi rotary evaporator with vacuum accessories, pressure filter. Magnetic stirrer, oil pump. |

### Reaction:

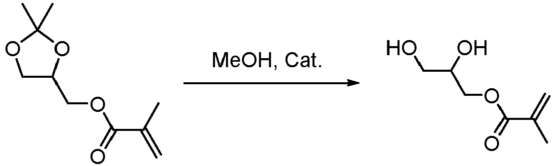

| | | | | |
|---|---|---|---|---|
| **Mixture:** | 50 | g | of 2,2-dimethyl-1,3-dioxolan-4-ylmethyl methacrylate (IPGMA) | = 0.25 mol |
| | 80 | g | of methanol | = 2.5 mol |
| | 0.016 | g | of hydroquinone monomethyl ether rel. to GMMA | = 400 ppm |
| | 0.004 | g | of hydroxy-2,2,6,6-tetramethylpiperidinooxyl (TEMPOL); rel. to GMMA | = 100 ppm |
| | 13 | g | of catalyst/Tonsil^{®} 312 FF standard rel. to mixture | = 10% |

### Procedure:

The batch is boiled at 65°C under reflux with stirring for 20 h. The GMMA crude ester obtained is filtered through a pressure filter with K800 filter layer. The filter is rinsed with ca. 100 g of methanol. The clear, pale yellow GMMA crude ester is freed of solvent at 50°C bath temperature at 20 mbar on a rotary evaporator for 30 min.

### Results:

| | **IPGMA mol** | **MeOH mol** | | | **Reaction** | | | | | | **GMMA GC-RV in area%** | | | |
|---|---|---|---|---|---|---|---|---|---|---|---|---|---|---|
| | | | **Catalyst** | **% by weight rel. to mixture** | **Duration h** | **Bottoms max. °C** | **Mass g = % of theory** | **MeOH** | **IPG** | **IPGMA** | **Glycerol** | **Glycerol dimethacrylate** | **GMMA** | **Remarks** |
| **1** | 0.25 | 2.5 | **Amberlyst^{®} A15 washed and dried LJ: 20896/57** | 10 | 20 | 50 | 43g = 38% | - | 9.3 | 0.4 | 16.4 | 16.5 | **35.9** | **Clear dark product** |
| **2** | 0.25 | 2.5 | **p-TS** | 1 | 22 | 50 | 43g = 8% | - | 26.5 | 2.9 | 25.2 | 8.9 | **7.8** | **Clear yellow product --> polymer** |
| **3** | 0.25 | 2.5 | **Montmorillonite K10** | 10 | 22 | 50 | 41g = 58% | 3.0 | 1.0 | 34.2 | 0.8 | 0.1 | **56.6** | **Clear yellow product** |
| **4** | 0.25 | 2.5 | **Montmorillonite K10** | 10 | 5 | 50 | 41g = 59% | 2.3 | 0.6 | 37.4 | 0.5 | < 0.1 | **58.0** | **Clear yellow product** |
| **5** | **0.25** | 2.5 | **Montmorillonite K30** | 10 | 5 | 65 | 42g = 69% | 0.1 | 1.1 | 26.1 | 1.6 | 0.1 | **65.3** | **Clear yellow product** |
| **6** | **0.25** | 2.5 | **Tonsil^{®} 312 FF** | 10 | 22 | 50 | 41g = 68% | 0.6 | 0.5 | 31.2 | 0.4 | - | **66.6** | **Clear pale yellow product** |
| **7** | 0.25 | 2.5 | **Tonsil^{®} 312 FF** | 10 | 64 | RT | 44g = 65% | 2.2 | 0.5 | 38.0 | 0.4 | - | **58.7** | **Clear pale yellow product** |
| **8** | 0.25 | 2.5 | **Tonsil^{®} 312 FF** | 10 | 20 | 65 | 43g = 79% | 2.3 | 0.5 | 22.3 | 0.6 | < 0.1 | **73.1** | **Clear pale yellow product** |

## Claims

1. A method for producing glycerol mono(meth)acrylate, **characterized in that** 2,2-dimethyl-1,3-dioxolan-4-yl methyl (meth)acrylate is reacted with methanol in the presence of an acidic silicate catalyst,
wherein the silicate is a sheet silicate or a mixture of sheet silicates selected from the group consisting of montmorillonite, kaolinite, hectorite, halloysite and bentonite, and
wherein the reaction is performed in anhydrous manner and **in that** the product is anhydrous glycerol mono(meth)acrylate.

2. Method according to Claim 1, **characterized in that** the sheet silicate or the mixture of sheet silicates have a specific surface area greater than 50 m²/g, preferably greater than 220 m²/g and particularly preferably greater than 320 m²/g.

3. Method according to Claims 1 or 2, **characterized in that** the sheet silicate or the mixture of sheet silicates is montmorillonite or bentonite.

4. Method according to any of the preceding claims, **characterized in that** the acidic catalyst is used in an amount from 1% by weight to 15% by weight, based on the reaction batch.

5. Method according to any of the preceding claims, **characterized in that** at least one stabilizer is used selected from the group consisting of octadecyl-3-(3,5-di-tert-butyl-4-hydroxyphenyl) propionate, phenothiazine, hydroquinone, hydroquinone monomethyl ether, 4-hydroxy-2,2,6,6-tetramethylpiperidinooxyl (TEMPOL), 2,4-dimethyl-6-tert-butylphenol, 2,6-di-tert-butylphenol, 2,6-di-tert-butyl-4-methylphenol, para-substituted phenylenediamines such as for example N,N'-diphenyl-p-phenylenediamine, 1,4-benzoquinone, 2,6-di-tert-butyl-alpha-(dimethylamino)-p-cresol, 2,5-di-tert-butylhydroquinone.

6. Method according to Claim 5, **characterized in that** the total amount of stabilizers used is between 0.001% by weight and 0.5% by weight.

7. Method according to either of Claims 5 and 6, **characterized in that** the stabilizers hydroquinone monomethyl ether and hydroxy-2,2,6,6-tetramethylpiperidinooxyl (TEMPOL) are used in combination.

8. Method according to Claim 7, **characterized in that** the ratio of hydroquinone monomethyl ether to TEMPOL is in the range between 10:1 and 4:1.

9. Method according to any of the preceding claims, **characterized in that** the reaction temperature is between 20°C and 80°C.

10. Method according to Claim 9, **characterized in that** the reaction temperature is between 40°C and 70°C.

## Patentansprüche

1. Verfahren zur Herstellung von Glycerinmono(meth)-acrylat, **dadurch gekennzeichnet, dass** 2,2-Dimethyl-1,3-dioxolan-4-ylmethyl-(meth)acrylat in Gegenwart eines sauren Silikatkatalysators mit Methanol umgesetzt wird,
wobei das Silikat ein Schichtsilikat oder ein Gemisch aus Schichtsilikaten ausgewählt aus der Gruppe bestehend aus Montmorillonit, Kaolinit, Hectorit, Halloysit und Bentonit ist und
wobei die Umsetzung wasserfrei durchgeführt wird, und es sich bei dem Produkt um wasserfreies Glycerinmono(meth)-acrylat handelt.

2. Verfahren nach Anspruch 1, **dadurch gekennzeichnet, dass** das Schichtsilikat bzw. das Gemisch aus Schichtsilikaten eine spezifische Oberfläche größer als 50 m²/g, vorzugsweise größer als 220 m²/g und besonders bevorzugt größer als 320 m²/g aufweist.

3. Verfahren nach Anspruch 1 oder 2, **dadurch gekennzeichnet, dass** das Schichtsilikat bzw. das Gemisch aus Schichtsilikaten Montmorillonit oder Bentonit ist.

4. Verfahren nach einem der vorhergehenden Ansprüche, **dadurch gekennzeichnet, dass** der saure Katalysator in einer Menge von 1 Gew.% bis 15 Gew.%, bezogen auf den Reaktionsansatz, eingesetzt wird.

5. Verfahren nach einem der vorhergehenden Ansprüche, **dadurch gekennzeichnet, dass** mindestens ein Stabilisator ausgewählt aus der Gruppe bestehend aus Octadecyl-3-(3,5-di-tert-butyl-4-hydroxyphenyl)propionat, Phenothiazin, Hydrochinon, Hydrochinonmonomethylether, 4-Hydroxy-2,2,6,6-tetramethylpiperidinooxyl (TEMPOL), 2,4-Dimethyl-6-tert-butylphenol, 2,6-Di-tert-butylphenol, 2,6-Di-tert-butyl-4-methylphenol, para-substituierten Phenylendiaminen wie z. B. N,N'-Diphenyl-p-phenylendiamin, 1,4-Benzochinon, 2,6-Di-tert-butyl-alpha-(dimethylamino)-p-cresol, 2,5-Di-tert-butylhydrochinon eingesetzt wird.

6. Verfahren nach Anspruch 5, **dadurch gekennzeichnet, dass** die Gesamtmenge an eingesetzten Stabilisatoren zwischen 0.001 Gew.% und 0.5 Gew.% liegt.

7. Verfahren nach einem der Ansprüche 5 und 6, **dadurch gekennzeichnet, dass** die Stabilisatoren Hydrochinonmonomethylether und Hydroxy-2,2,6,6-tetramethylpiperidino-oxyl (TEMPOL) in Kombination verwendet werden.

8. Verfahren nach Anspruch 7, **dadurch gekennzeichnet, dass** das Verhältnis von Hydrochinonmonomethylether zu TEMPOL im Bereich zwischen 10:1 und 4:1 liegt.

9. Verfahren nach einem der vorhergehenden Ansprüche, **dadurch gekennzeichnet, dass** die Reaktionstemperatur zwischen 20°C und 80°C liegt.

10. Verfahren nach Anspruch 9, **dadurch gekennzeichnet, dass** die Reaktionstemperatur zwischen 40°C und 70°C liegt.

## Revendications

1. Procédé de production de mono(méth)acrylate de glycérol, **caractérisé en ce que** du (méth)acrylate de 2,2-diméthyl-1,3-dioxolan-4-yle est amené à réagir avec du méthanol en présence d'un catalyseur silicate acide,
dans lequel le silicate est un phyllosilicate ou un mélange de phyllosilicates choisis dans le groupe constitué par la montmorillonite, la kaolinite, l'hectorite, l'halloysite et la bentonite et
dans lequel la réaction est effectuée d'une manière anhydre et **en ce que** le produit est du mono (méth) acrylate de glycérol anhydre.

2. Procédé selon la revendication 1, **caractérisé en ce que** le phyllosilicate ou le mélange de phyllosilicates ont une surface spécifique supérieure à 50 m²/g, de préférence supérieure à 220 m²/g et particulièrement de préférence supérieure à 320 m²/g.

3. Procédé selon les revendications 1 ou 2, **caractérisé en ce que** le phyllosilicate ou le mélange de phyllosilicates est de la montmorillonite ou de la bentonite.

4. Procédé selon l'une quelconque des revendications précédentes, **caractérisé en ce que** le catalyseur acide est utilisé en une quantité allant de 1 % en poids à 15 % en poids, sur la base du lot de réaction.

5. Procédé selon l'une quelconque des revendications précédentes, **caractérisé en ce qu'**au moins un stabilisant est utilisé choisi dans le groupe constitué par le propionate d'octadécyl-3-(3,5-di-tert-butyl-4-hydroxyphényle), la phénothiazine, l'hydroquinone, l'éther monométhylique d'hydroquinone, le 4-hydroxy-2,2,6,6-tétraméthylpipéridinooxyle (TEMPOL), le 2,4-diméthyl-6-*tert*-butylphénol, le 2,6-di-*tert*-butylphénol, le 2,6-di-*tert*-butyl-4-méthylphénol, des *para-* phénylènediamines substituées telles que par exemple la *N*,*N*'-diphényl-*p*-phénylènediamine, la 1,4-benzoquinone, le 2,6-di-*tert*-butyl-alpha-(diméthylamino)-*p*-crésol, la 2,5-di-*tert*-butylhydroquinone.

6. Procédé selon la revendication 5, **caractérisé en ce que** la quantité totale de stabilisants utilisés est comprise entre 0,001 % en poids et 0,5 % en poids.

7. Procédé selon l'une ou l'autre des revendications 5 et 6, **caractérisé en ce que** les stabilisants éther monométhylique d'hydroquinone et hydroxy-2,2,6,6-tétraméthylpipéridinooxyle (TEMPOL) sont utilisés en association.

8. Procédé selon la revendication 7, **caractérisé en ce que** le rapport de l'éther monométhylique d'hydroquinone au TEMPOL est dans la plage comprise entre 10:1 et 4:1.

9. Procédé selon l'une quelconque des revendications précédentes, **caractérisé en ce que** la température de réaction est comprise entre 20 °C et 80 °C.

10. Procédé selon la revendication 9, **caractérisé en ce que** la température de réaction est comprise entre 40 °C et 70 °C.
